# EUROPEAN PATENT SPECIFICATION

(11) **EP 4 282 374 B1**
(45) Date of publication and mention of the grant of the patent: **25.09.2024**
(21) Application number: 23174839.3
(22) Date of filing: 23.05.2023
(51) Int. Cl.: A61C 7/00, A61C 7/20, A61C 7/12, A61C 7/14

(54) **METHOD OF GENERATING BENDING PATH OF TOOTH STRAIGHTENING WIRE AND APPARATUS FOR PERFORMING THE METHOD**
VERFAHREN ZUR ERZEUGUNG EINES BIEGEWEGES VON ZAHNREGULIERUNGSDRÄHTEN UND VORRICHTUNG ZUR DURCHFÜHRUNG DES VERFAHRENS
PROCÉDÉ DE GÉNÉRATION DE TRAJET DE PLIAGE DE FIL DE REDRESSEMENT DE DENT ET APPAREIL POUR RÉALISER LE PROCÉDÉ

(30) Priority: 23.05.2022 KR 20220063094
(43) Date of publication of application: 29.11.2023
(73) Proprietor: Yoat Corporation, Seoul 08513 (KR)
(72) Inventor: JUNG, Youn Ho, 08513 Seoul (KR)
(74) Representative: Isarpatent

(56) References cited:
- WO-A1-2021/242050
- US-A1- 2005 043 837
- US-A1- 2020 281 702

## Description

### BACKGROUND

### 1. Field

Embodiments of the present disclosure relate to a technique for generating a bending path of a tooth straightening wire.

### 2. Discussion of Related Art

Dental treatment is a series of treatments for preventing, diagnosing, and treating diseases or abnormal conditions in the facial region including the teeth and surrounding tissues and the oral cavity.

Recently, many orthodontic treatments have been performed in addition to treatments necessary to improve oral hygiene and to maintain healthy teeth. Orthodontic treatment involves not only simply straightening crooked teeth, but also creating healthy oral tissues and beautiful facial features by correcting various skeletal inconsistencies that may occur during growth so that they can function normally.

A method in which orthodontic treatment is performed using a wire after an orthodontic bracket is mounted on teeth is widely used as an orthodontic treatment method. In this orthodontic treatment method, the wire is replaced according to an orthodontic treatment process.

However, when an oral cavity is scanned using an intraoral 3D scanner, the orthodontic bracket mounted on the teeth during orthodontic treatment is not accurately scanned. That is, there is a problem in that it is difficult to accurately check a position of a groove through which a wire is inserted into an orthodontic bracket in an image scanned using an intraoral 3D scanner.

Therefore, when a scanned image is used, there is a problem in that it is not possible to accurately bend the wire for insertion into the orthodontic bracket.

US 2005/043837 discloses an interactive orthodontic care system based on intra-oral scanning of teeth.

### SUMMARY

The invention is as defined in the appended claims.

Embodiments of the present disclosure are intended to correct an image by matching a pre-stored orthodontic bracket image with scan data obtained using an intraoral 3D scanner.

Further, embodiments of the present invention are intended to generate a bending path of a tooth straightening wire through a corrected image.

According to an aspect of the present disclosure, there is provided a computing device which includes one or more processors, and a memory configured to store one or more programs executed by the one or more processors, including an image acquisition part configured to acquire an oral image of an inside of a target patient's oral cavity using an intraoral 3D scanner, an image analyzer configured to generate a tooth image and an image of a bracket attached to a plurality of teeth through the obtained oral image, an image corrector configured to match the image of the attached bracket and an orthodontic bracket image included in pre-stored orthodontic bracket information and to generate a correction image for the oral image based on the tooth image and the matching orthodontic bracket image, and a path generator configured to generate a bending path of a wire based on the correction image.

The path generator may calculate a position of an orthodontic bracket groove in the correction image based on the matching orthodontic bracket information and may generate a virtual point at the calculated position of the orthodontic bracket groove to generate the bending path of the wire.

The bending path of the wire may generate at least two or more virtual points for each tooth, and the virtual points may be two or more points generated in left and right boundaries of the calculated position of the orthodontic bracket groove.

The image analyzer may generate an image of each tooth in the oral cavity based on the oral image, generate an image of a bracket attached on each tooth, and generate position information of the bracket attached to each tooth based on an adhesive surface of the attached bracket.

The image corrector may compare the image of the attached bracket with the pre-stored orthodontic bracket image and may extract an orthodontic bracket image that matches the image of the attached bracket in the pre-stored orthodontic bracket image.

The image corrector may place the matching orthodontic bracket image in the same portion as the image of the bracket attached to each tooth on the basis of the position information of the attached bracket.

The image corrector may match the matching orthodontic bracket image and the image of the attached bracket using an iterative closest point (ICP) algorithm.

According to another aspect of the present disclosure, there is provided a method of generating a bending path of a tooth straightening wire, which is performed in a computing device including one or more processors, and a memory for storing one or more programs executed by the one or more processors, including obtaining an oral image of an inside of a target patient's oral cavity using an intraoral 3D scanner, generating a tooth image and an image of a bracket attached to a plurality of teeth through the obtained oral image, matching the image of the attached bracket and an orthodontic bracket image included in pre-stored orthodontic bracket information and generating a correction image for the oral image based on the tooth image and the matching orthodontic bracket image, and generating a bending path of a wire based on the correction image.

The generating of the bending path of the wire may further include calculating a position of an orthodontic bracket groove in the correction image based on the matching correction bracket information, generating a virtual point at the calculated position of the orthodontic bracket groove to generate the bending path of the wire, and outputting the generated bending path of the wire.

The bending path of the wire may generate at least two or more virtual points for each tooth, and the virtual points may be two or more points generated in left and right boundaries of the calculated position of the orthodontic bracket groove.

The generating of the tooth image and the image of the bracket attached to the plurality of teeth may further include generating an image of each tooth in the oral cavity based on the oral image, generating an image of a bracket attached to each of the teeth, and generating position information of the bracket attached to each tooth based on an adhesive surface of the attached bracket.

The generating of the correction image may further include comparing the image of the attached bracket with the pre-stored orthodontic bracket image, and extracting an orthodontic bracket image that matches the image of the attached bracket in the pre-stored orthodontic bracket image.

The generating of the correction image may further include placing the matching orthodontic bracket image in the same portion as the image of the bracket attached to each tooth on the basis of the position information of the attached bracket.

According to the embodiments of the present disclosure, it is possible to obtain an accurate position of a bracket groove from scan data by matching the scan data obtained using an intraoral 3D scanner and a pre-stored orthodontic bracket image to correct an image.

Further, according to the embodiments of the present disclosure, an accurate bending path can be generated by generating a bending path of a tooth straightening wire through a corrected image, and a wire can be formed simply and accurately according to the wire bending path.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:
FIG. 1 is a block diagram illustrating an apparatus for generating a bending path of a tooth straightening wire according to an embodiment of the present disclosure;
FIG. 2 is a view for describing a method of adjusting a position of a virtual 3D tooth model according to an embodiment of the present disclosure;
FIGS. 3 and 4 are diagrams illustrating a process of generating a correction image according to an embodiment of the present disclosure;
FIG. 5 is a flowchart illustrating a path of a wire according to an embodiment of the present disclosure;
FIG. 6 is a flowchart for describing a method of generating a bending path of a tooth straightening wire according to an embodiment of the present invention; and
FIG. 7 is a block diagram illustrating and describing a computing environment including a computing device suitable for use in exemplary embodiments.

### DETAILED DESCRIPTION OF EXEMPLARY EMBODIMENTS

Hereinafter, specific embodiments of the present disclosure will be described in detail with reference to the accompanying drawings. The following detailed description is provided to assist the reader in gaining a comprehensive understanding of the methods, apparatuses, and/or systems described herein. However, the description is only exemplary, and the present disclosure is not limited thereto.

In describing embodiments of the present disclosure, when it is determined that detailed description of known techniques associated with the present disclosure would unnecessarily obscure the subject matter of the present disclosure, the detailed description thereof will be omitted. Also, terms used herein are defined in consideration of the functions of the present disclosure, and may be changed depending on the intent or custom of a user or operator. Accordingly, the terms should be defined based on the following overall description of this specification. The terminology used herein is only for the purpose of describing embodiments of the present disclosure and is not restrictive. The singular forms "a" and "an" are intended to include the plural forms as well, unless the context clearly indicates otherwise. It should be understood that the terms "comprises," "comprising," "includes," and/or "including" specify the presence of stated features, integers, steps, operations, elements, and/or components when used herein, but do not preclude the presence or addition of one or more other features, integers, steps, operations, elements, components, and/or groups thereof.

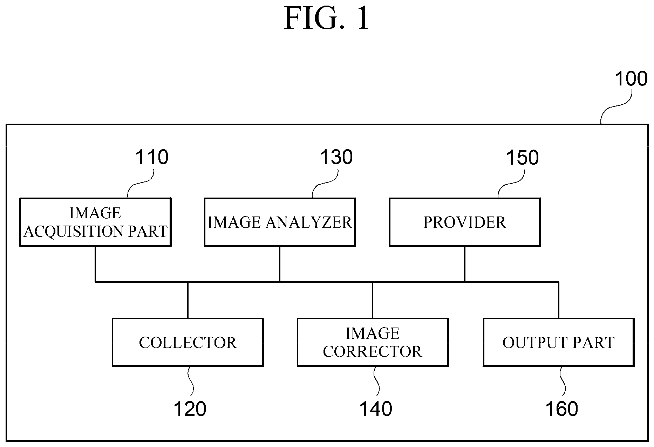

FIG. 1 is a block diagram illustrating an apparatus for generating a bending path of a tooth straightening wire according to an embodiment of the present disclosure.

As illustrated in FIG. 1, the apparatus 100 for generating a bending path of a tooth straightening wire according to the embodiment of the present disclosure may include an image acquisition part 110, a collector 120, an image analyzer 130, an image corrector 140, a path generator 150, and an output part 160.

The image acquisition part 110 may acquire an oral image (scan data) of the inside of an oral cavity of a target patient using an intraoral 3D scanner. Specifically, the image acquisition part 110 may acquire an oral image of the target patient by inserting the intraoral 3D scanner into the oral cavity of the target patient and scanning the inside of the oral cavity. At this time, data acquired by the intraoral 3D scanner may be acquired by a computer and peripheral devices connected to the computer and then may be recorded in a memory device, for example, may have a structure that is generated and stored as a 3D stereo-lithography (STL) file. Meanwhile, in the present disclosure, the intraoral 3D scanner is used as a 3D scanning device, but the present disclosure is not limited thereto, and various scanning devices such as a coordinate measuring machine (CMM), a laser scanner, and an optical scanner may be used.

The collector 120 may receive orthodontic bracket information from an external server (for example, a server of a bracket manufacturer). Here, the orthodontic bracket information may include image, material, bracket groove information (a shape, a position, or the like of a groove formed in the bracket to which the wire is coupled), and the like of the orthodontic bracket for each manufacturer. Meanwhile, here, although the orthodontic bracket information is received from the external server in the example described here, the present disclosure is not limited thereto, and the orthodontic bracket information may be stored in advance. Here, the orthodontic bracket is a device attached to a tooth, may be firmly attached to a surface of the orthodontic target tooth for orthodontic treatment, and may be interconnected using a wire. The orthodontic bracket may be formed with a bracket groove to be coupled with the wire. Thus, the orthodontic target tooth can be gradually moved by adjusting tension applied to the wire and adjusting a direction and magnitude of a force applied to the wire. Thus, a position and posture of the orthodontic target tooth are changed by the tension of the wire, and the orthodontic treatment is performed while the tooth is moved little by little.

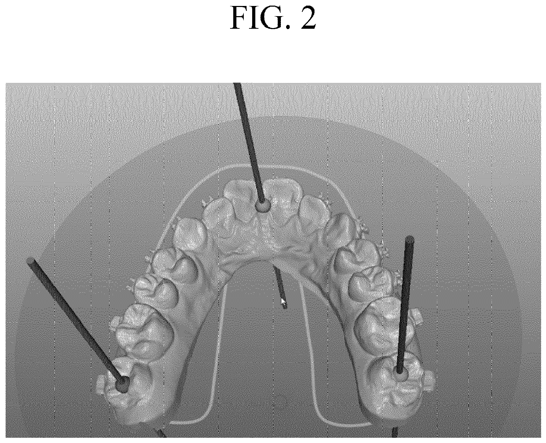

The image analyzer 130 may analyze the oral image (the scan data) obtained from the image acquisition part 110 and may generate a tooth image and an image of the bracket attached to a plurality of teeth. Here, the images may be 3D images obtained from the scan data. For example, as illustrated in FIG. 2, the scan data generated and stored as an STL file is loaded by a computer and forms a virtual 3D dental model, and the virtual 3D dental model may be generated for each of an upper jaw and a lower jaw of the plurality of teeth. The upper jaw and lower jaw in which the virtual 3D dental model is formed may form any three axes with respect to the virtual 3D dental model, and a user may move and rotate the axes to arrange the upper jaw and the lower jaw in positions desired by the user.

In an exemplary embodiment, the image analyzer 130 may extract each of the tooth images based on the oral image and may extract the image of the bracket attached to each tooth. Also, the image analyzer 130 may generate position information of the attached brackets attached to each tooth based on an adhesive surface of the attached brackets (the brackets attached to the teeth). Here, the position information of the attached bracket may include an attachment surface of the tooth to which the attachment surface of the bracket is attached, and position coordinates of the bracket attached on the attachment surface of the tooth.

The image corrector 140 may generate a correction image based on the position information of the orthodontic bracket image, the tooth image, and the position information of the attached bracket that match the attached bracket image among the pre-stored orthodontic bracket images. Here, each of the tooth images may be changed by adjusting the left side, the right side, the front side, the back side, and a height according to necessity (progress of the orthodontic treatment).

In an exemplary embodiment, the image corrector 140 may compare a pre-stored orthodontic bracket image with an attached bracket image and may extract an orthodontic bracket image that matches the attached bracket image in the pre-stored orthodontic bracket information. In addition, the image corrector 140 may generate a correction image by combining the matching orthodontic bracket image based on the position information of the attached bracket with each of the tooth images. Here, the position information of the attached bracket may include position coordinates of the attachment surface of the tooth to which the attachment surface of the bracket is attached and the bracket attached to the attachment surface of the tooth. That is, the image corrector 140 may place the matching orthodontic bracket image based on the position information of the attached bracket in the same portion as the attached bracket image of each of the tooth images. At this time, the image corrector 140 may minimize a distance error between feature points of the matching orthodontic bracket image and feature points of the attached bracket image in order to match the matching orthodontic bracket image and the attached bracket image placed in the same portion in each of the tooth images. For example, the image corrector 140 may calculate a translation matrix and a rotation matrix using an iterative closest point (ICP) algorithm so that the distance error between feature points of the orthodontic bracket image and feature points of the corresponding attached bracket image can be minimized. In addition, the image corrector 140 may apply the calculated translation matrix and rotation matrix to the feature points of the matching orthodontic bracket image to match the matching orthodontic bracket image and the attached bracket image.

That is, due to the matching orthodontic bracket image based on the position information of the attached bracket being located in the same portion as the attached bracket image of each of the tooth images and the matching orthodontic bracket image and the attached bracket image matching each other using the ICP algorithm, accuracy of the ICP algorithm can be increased by giving an appropriate initial position of the two images (that is, a high degree of overlap between the matching orthodontic bracket image and the attached bracket image of the tooth image). Accordingly, the image corrector 140 may change an existing attached bracket image to the matching orthodontic bracket image.

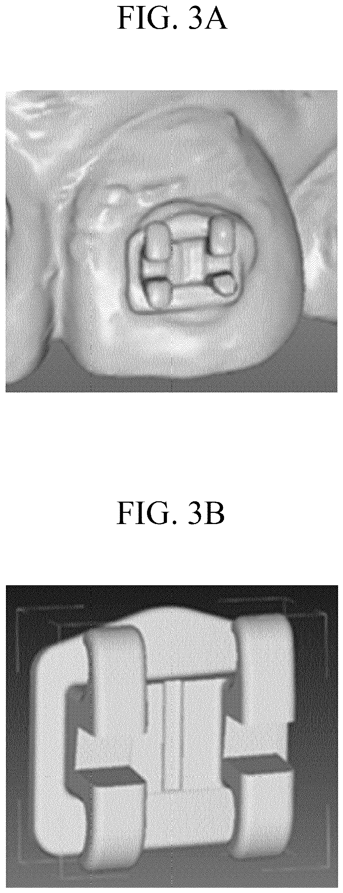

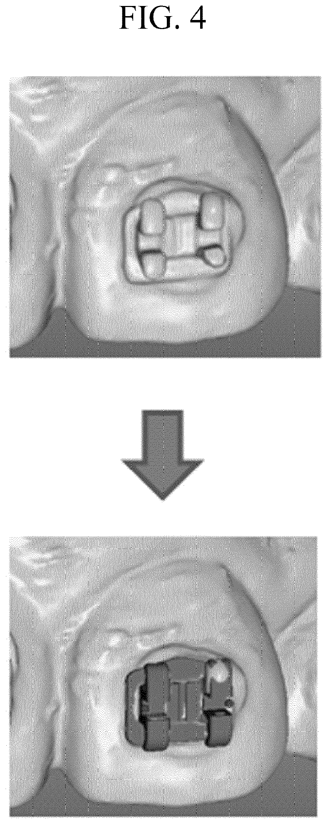

For example, as shown in FIG. 3, FIG. 3A is an attached bracket image obtained from an oral image, and FIG. 3B is a pre-stored orthodontic bracket image. Since it is difficult to accurately identify a position of the bracket groove of the orthodontic bracket in the oral image, it is possible to extract the orthodontic bracket image that matches the attached bracket image in the pre-stored orthodontic bracket information. In addition, as shown in FIG. 4, it is possible to change the attached bracket image on the tooth to the matching orthodontic bracket image using the matching orthodontic bracket image. Thus, it is possible to obtain the accurate position of the bracket groove from the oral image.

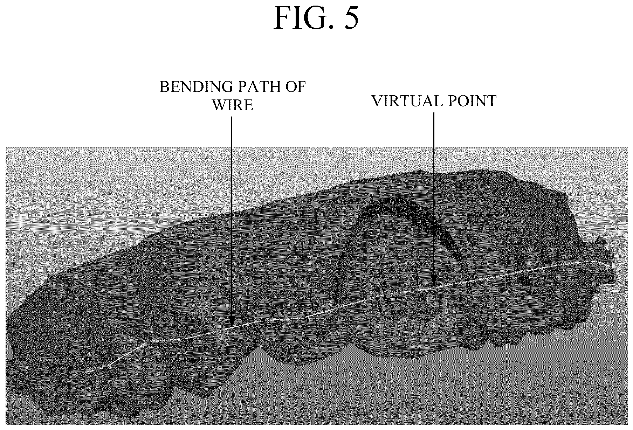

The path generator 150 may calculate a position of a bracket groove in the correction image based on the bracket groove information of the matching orthodontic bracket, and may generate a virtual point at the calculated position of the bracket groove to generate a bending path of the wire. At this time, at least two or more virtual points may be generated for each of the orthodontic brackets. Specifically, the path generator 150 may generate the virtual points on each of left and right boundaries of the calculated position of the bracket groove. Also, the path generator 150 may set a bending angle at each of the virtual points and may connect each of the virtual points based on the bending angle to generate the bending path of the wire. For example, as shown in FIG. 5, the path generator 150 may generate a bending path of the wire that connects each of the virtual points generated in the groove of the orthodontic bracket. Here, the bending angle may be set in consideration of a position and posture of an orthodontic target tooth according to an orthodontic treatment process. At this time, the bending path of the wire can be changed by adjusting the left, right, front, back, and height positions with respect to each of the virtual points as needed.

The output part 160 may output the generated bending path of the wire. For example, the bending path of the wire output from the output part 160 may be data generated and stored as a comma-separated values (CSV) file. In addition, the output part 160 may output the bending path so that a thickness thereof differs according to the type of wire used when the bending path of the wire is output. That is, output data for the bending path of the wire may be adjusted in a form generated on a line connecting a center point of a vertical cross-section of the wire in consideration of a thickness of the wire.

Meanwhile, the output data on the bending path of the wire output from the output part 160 is input to a bending machine, and the bending machine may bend the wire according to the output data on the bending path of the wire.

Accordingly, the apparatus for generating a bending path of a tooth straightening wire according to the embodiment of the present disclosure can obtain an accurate position of the bracket groove from the scan data by matching the pre-stored orthodontic bracket image and the scan data obtained using the intraoral 3D scanner and correcting the image.

Further, the apparatus for generating a bending path of a tooth straightening wire according to the embodiment of the present invention can generate an accurate bending path by generating a bending path of a tooth straightening wire through a corrected image and can form a wire simply and accurately according to the bending path of the wire.

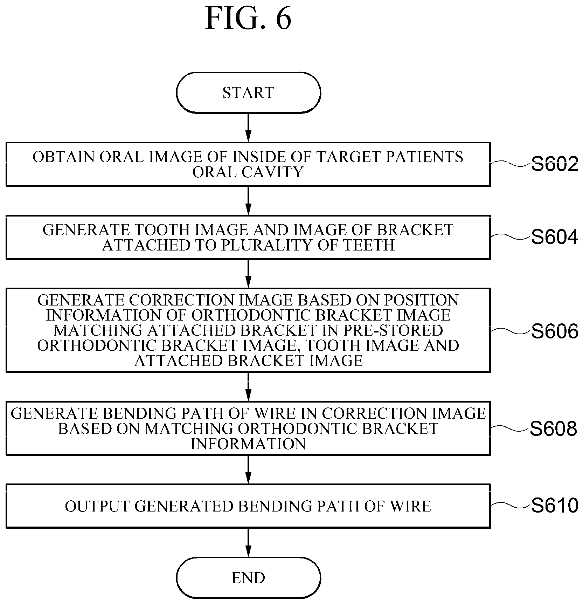

FIG. 6 is a flowchart for describing a method of generating a bending path of a tooth straightening wire according to an embodiment of the present disclosure. The correction image generation method for orthodontic treatment according to the embodiment of the present disclosure may be performed in a computing device 12 including one or more processors and one or more memories that store one or more programs executed by the one or more processors. To this end, the correction image generation method for orthodontic treatment may be implemented in the form of a program or software including one or more computer-executable instructions and may be stored in the memory.

In addition, although the method has been described by dividing the method into a plurality of steps in the illustrated flowchart, at least some of the steps may be performed in a reversed order, may be performed together in combination with other steps, may be omitted, may be divided into detailed steps, or may be performed by adding one or more steps that are not shown.

First, the computing device 12 obtains the scan data (the oral image) of the inside of a target patient's oral cavity using an intraoral 3D scanner (S602). Specifically, the computing device 12 may acquire the oral image of the target patient by inserting the intraoral 3D scanner into the oral cavity of the target patient and scanning the inside of the oral cavity.

Next, the computing device 12 analyzes the oral image to generate a tooth image and an image of a bracket attached to a plurality of teeth (S604). Here, the image may be a 3D image obtained from the scan data (the oral image). Specifically, the computing device 12 may generate an image of each tooth based on the oral image and may generate the image of the bracket attached to each tooth. Also, the computing device 12 may generate position information of the attached bracket attached to each tooth based on the adhesive surface of each of the attached brackets (each of the brackets attached to the teeth). Here, the position information of the attached bracket may include position coordinates of the attachment surface of the tooth to which the attachment surface of the bracket is attached, and position coordinates of the bracket attached to the attachment surface of the tooth.

Then, the computing device 12 matches the pre-stored orthodontic bracket image and the attached bracket image and generates a correction image based on the tooth image, the matching orthodontic bracket image, and the position information of the attached bracket (S606). Specifically, the computing device 12 may compare the pre-stored orthodontic bracket image with the attached bracket image and may extract an orthodontic bracket image that matches the attached bracket image in the pre-stored orthodontic bracket information. In addition, the computing device 12 may generate a correction image by combining the matching orthodontic bracket image based on the position information of the attached bracket with each of the tooth images.

Then, the computing device 12 may calculate the position of the bracket groove in the correction image based on the bracket groove information of the matching orthodontic bracket and generate a virtual point at the calculated position of the bracket groove to generate the bending path of the wire (S608).

Finally, the computing device 12 outputs the generated bending path of the wire (S610). For example, the output bending path of the wire may be data generated and stored as a CSV file. In this case, output data on the bending path of the wire output from the computing device 12 is input to a bending machine, and the bending machine may bend the wire according to the output data on the bending path of the wire.

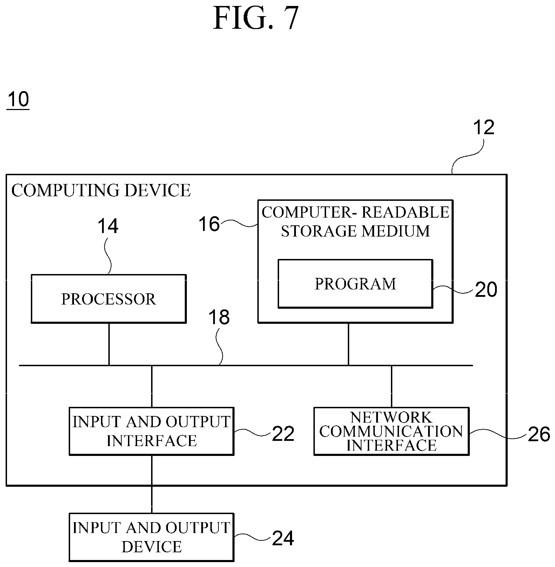

FIG. 7 is a block diagram illustrating and describing a computing environment including a computing device suitable for use in exemplary embodiments. In the illustrated embodiment, each component may have functions and capabilities other than those which will be described below and may include additional components in addition to those which will be described below.

The illustrated computing environment 10 includes the computing device 12. In one embodiment, the computing device 12 may be a device for generating a bending path of a tooth straightening wire.

The computing device 12 includes at least one processor 14, a computer-readable storage medium 16, and a communication bus 18. The processor 14 may be operated by the computing device 12 in accordance with the exemplary embodiments discussed above. For example, the processor 14 may execute one or more programs stored in the computer-readable storage medium 16. The one or more programs may include one or more computer-executable instructions, and the computer-executable instructions may be configured so that the computing device 12 performs operations in accordance with the exemplary embodiment when the instructions are executed by the processor 14.

The computer-readable storage medium 16 is configured to store computer-executable instructions or program codes, program data, and/or other suitable types of information. The program 20 stored in the computer-readable storage medium 16 includes a set of instructions executable by the processor 14. In one embodiment, the computer-readable storage medium 16 may be a memory (a volatile memory such as a random access memory, a non-volatile memory, or a suitable combination thereof), one or more magnetic disk storage devices, optical disc storage devices, flash memory devices, other types of storage media that can be accessed by the computing device 12 and can store desired information, or a suitable combination thereof.

The communication bus 18 interconnects various other components of the computing device 12 including the processor 14 and the computer-readable storage medium 16.

The computing device 12 may also include one or more input and output interfaces 22 that provide interfaces for one or more input and output devices 24, and one or more network communication interfaces 26. The input and output interfaces 22 and the network communication interfaces 26 are connected to the communication bus 18. The input and output devices 24 may be connected to other components of the computing device 12 via the input and output interfaces 22. The exemplary input and output devices 24 may include a pointing device (such as a mouse or track pad), a keyboard, a touch input device (such as a touch pad or touch screen), a voice or sound input device, various types of input devices such as sensor devices and/or imaging devices, and/or output devices such as display devices, printers, speakers, and/or network cards. The exemplary input and output devices 24 may be included in the computing device 12 as a component constituting the computing device 12 and may be connected to the computing device 12 as a separate device distinct from the computing device 12.

Although example embodiments of the present disclosure have been described in detail, it should be understood by those skilled in the art that various changes may be made without departing from the scope of the present disclosure. Therefore, the scope of the present invention is to be determined by the following claims and is not restricted or limited by the foregoing detailed description.

## Claims

1. A computing device (12) which includes one or more processors (14), and a memory configured to store one or more programs executed by the one or more processors (14), the computing device (12) comprising:
an image acquisition part (110) configured to acquire an oral image of an inside of a target patient's oral cavity using an intraoral 3D scanner;
an image analyzer (130) configured to generate a tooth image and an image of a bracket attached to a plurality of teeth through the obtained oral image;
an image corrector (140) configured to match the image of the attached bracket and an orthodontic bracket image included in pre-stored orthodontic bracket information and to generate a correction image for the oral image based on the tooth image and the matching orthodontic bracket image; and
a path generator (150) configured to generate a bending path of a wire based on the correction image.

2. The computing device of claim 1, wherein the path generator calculates a position of an orthodontic bracket groove in the correction image based on the matching orthodontic bracket information and generates a virtual point at the calculated position of the orthodontic bracket groove to generate the bending path of the wire.

3. The computing device of claim 2, wherein the bending path of the wire generates at least two or more virtual points for each tooth, and
the virtual points are two or more points generated in left and right boundaries of the calculated position of the orthodontic bracket groove.

4. The computing device of claim 1, wherein the image analyzer generates an image of each tooth in the oral cavity based on the oral image, generates an image of a bracket attached to each tooth, and generates position information of the bracket attached to each tooth based on an adhesive surface of the attached bracket.

5. The computing device of claim 4, wherein the image corrector compares the image of the attached bracket with the pre-stored orthodontic bracket image and extracts an orthodontic bracket image that matches the image of the attached bracket in the pre-stored orthodontic bracket image.

6. The computing device of claim 5, wherein the image corrector places the matching orthodontic bracket image in the same portion as the image of the bracket attached to each tooth on the basis of the position information of the attached bracket.

7. The computing device of claim 6, wherein the image corrector matches the matching orthodontic bracket image and the image of the attached bracket using an iterative closest point (ICP) algorithm.

8. A method of generating a bending path of a tooth straightening wire, which is performed in a computing device (12) including one or more processors (14) and a memory for storing one or more programs executed by the one or more processors (14) the method comprising:
obtaining (S602) an oral image of an inside of a target patient's oral cavity using an intraoral 3D scanner;
generating (S604) a tooth image and an image of a bracket attached to a plurality of teeth through the obtained oral image;
matching (S606) the image of the attached bracket and an orthodontic bracket image included in a pre-stored orthodontic bracket information and generating a correction image for the oral image based on the tooth image and the matching orthodontic bracket image; and
generating (S608) a bending path of a wire based on the correction image.

9. The method of claim 8, wherein the generating of the bending path of the wire further includes:
calculating a position of an orthodontic bracket groove in the correction image based on the matching correction bracket information;
generating a virtual point at the calculated position of the orthodontic bracket groove to generate the bending path of the wire; and
outputting the generated bending path of the wire.

10. The method of claim 9, wherein the bending path of the wire generates at least two or more virtual points for each tooth, and
the virtual points are two or more points generated in left and right boundaries of the calculated position of the orthodontic bracket groove.

11. The method of claim 8, wherein the generating of the tooth image and the image of the bracket attached to the plurality of teeth further includes:
generating an image of each tooth in the oral cavity based on the oral image;
generating an image of a bracket attached to each of the teeth; and
generating position information of the bracket attached to each tooth based on an adhesive surface of the attached bracket.

12. The method of claim 11, wherein the generating of the correction image further includes:
comparing the image of the attached bracket with the pre-stored orthodontic bracket image; and
extracting an orthodontic bracket image that matches the image of the attached bracket in the pre-stored orthodontic bracket image.

13. The method of claim 12, wherein the generating of the correction image further includes placing the matching orthodontic bracket image in the same portion as the image of the bracket attached to each tooth on the basis of the position information of the attached bracket.

## Patentansprüche

1. Computervorrichtung (12), aufweisend einen oder mehrere Prozessoren (14) und einen Speicher, der dafür eingerichtet ist, ein oder mehrere Programme zu speichern, die durch den einen oder die mehreren Prozessoren ausgeführt werden, wobei die Computervorrichtung (12) Folgendes umfasst:
einen Bildaufnahmeteil (110), der dafür eingerichtet ist, ein orales Bild eines Inneren der Mundhöhle eines Zielpatienten unter Verwendung eines intraoralen 3D-Scanners aufzunehmen;
einen Bildanalysator (130), der dafür eingerichtet ist, ein Zahnbild und ein Bild eines an mehreren Zähnen angebrachten Halteteils durch das erhaltene Mundbild zu generieren; und
eine Bildkorrekturvorrichtung (140), die dafür eingerichtet ist, das Bild des angebrachten Halteteils und ein orthodontisches Halteteilbild, das in vorab gespeicherten orthodontischen Halteteilinformationen enthalten ist, abzugleichen und ein Korrekturbild für das orale Bild auf der Grundlage des Zahnbildes und des übereinstimmenden orthodontischen Halteteilbildes zu generieren; und
einen Pfadgenerator (150), der dafür eingerichtet ist, auf der Grundlage des Korrekturbildes einen Biegepfad eines Drahtes zu generieren.

2. Computervorrichtung nach Anspruch 1, wobei der Pfadgenerator eine Position einer orthodontischen Halteteilnut in dem Korrekturbild auf der Grundlage der übereinstimmenden orthodontischen Halteteilinformationen berechnet und einen virtuellen Punkt an der berechneten Position der orthodontischen Halteteilnut generiert, um den Biegepfad des Drahtes zu generieren.

3. Computervorrichtung nach Anspruch 2, wobei der Biegepfad des Drahtes mindestens zwei oder mehr virtuelle Punkte für jeden Zahn generiert und
die virtuellen Punkte zwei oder mehr Punkte sind, die an der linken und der rechten Grenze der berechneten Position der orthodontischen Halteteilnut generiert werden.

4. Computervorrichtung nach Anspruch 1, wobei der Bildanalysator ein Bild jedes Zahns in der Mundhöhle auf der Grundlage des oralen Bildes generiert, ein Bild eines an jedem Zahn angebrachten Halteteils generiert und Positionsinformationen des an jedem Zahn angebrachten Halteteils auf der Grundlage einer Klebefläche des angebrachten Halteteils generiert.

5. Computervorrichtung nach Anspruch 4, wobei die Bildkorrekturvorrichtung das Bild des angebrachten Halteteils mit dem vorab gespeicherten orthodontischen Halteteilbild vergleicht und ein orthodontisches Halteteilbild extrahiert, das mit dem Bild des angebrachten Halteteils in dem vorab gespeicherten orthodontischen Halteteilbild übereinstimmt.

6. Computervorrichtung nach Anspruch 5, wobei die Bildkorrekturvorrichtung das übereinstimmende orthodontische Halteteilbild auf der Grundlage der Positionsinformationen des angebrachten Halteteils im selben Abschnitt wie das Bild des an jedem Zahn angebrachten Halteteils platziert.

7. Computervorrichtung nach Anspruch 6, wobei die Bildkorrekturvorrichtung das übereinstimmende orthodontische Halteteilbild und das Bild des angebrachten Halteteils unter Verwendung eines Iterative Closest Point (IC)-Algorithmus abgleicht.

8. Verfahren zum Generieren eines Biegepfades eines Zahnstellungskorrekturdrahtes, das in einer Computervorrichtung (12) durchgeführt wird, die einen oder mehrere Prozessoren (14) und einen Speicher zum Speichern eines oder mehrerer Programme aufweist, die durch den einen oder die mehreren Prozessoren ausgeführt werden, wobei das Verfahren umfasst:
Erhalten (S602) eines oralen Bildes eines Inneren der Mundhöhle eines Zielpatienten unter Verwendung eines intraoralen 3D-Scanners;
Generieren (S604) eines Zahnbildes und eines Bildes eines an mehreren Zähnen angebrachten Halteteils durch das erhaltene Mundbild;
Abgleichen (S606) des Bildes des angebrachten Halteteils und eines orthodontischen Halteteilbildes, das in vorab gespeicherten orthodontischen Halteteilinformationen enthalten ist, und Generieren eines Korrekturbildes für das orale Bild auf der Grundlage des Zahnbildes und des übereinstimmenden orthodontischen Halteteilbildes; und
Generieren (S608) eines Biegepfades eines Drahtes auf der Grundlage des Korrekturbildes.

9. Verfahren nach Anspruch 8, wobei das Generieren des Biegepfades des Drahtes des Weiteren umfasst:
Berechnen einer Position einer orthodontischen Halteteilnut in dem Korrekturbild auf der Grundlage der übereinstimmenden Korrekturhalteteilinformationen;
Generieren eines virtuellen Punktes an der berechneten Position der orthodontischen Halteteilnut, um den Biegepfad des Drahtes zu generieren; und
Ausgeben des generierten Biegepfades des Drahtes.

10. Verfahren nach Anspruch 9, wobei der Biegepfad des Drahtes mindestens zwei oder mehr virtuelle Punkte für jeden Zahn generiert und
die virtuellen Punkte zwei oder mehr Punkte sind, die an der linken und der rechten Grenze der berechneten Position der orthodontischen Halteteilnut generiert werden.

11. Verfahren nach Anspruch 8, wobei das Generieren des Zahnbildes und des Bildes des an den mehreren Zähnen angebrachten Halteteils des Weiteren umfasst:
Generieren eines Bildes jedes Zahns in der Mundhöhle auf der Grundlage des oralen Bildes;
Generieren eines Bildes eines an jedem der Zähne angebrachten Halteteils; und
Generieren von Positionsinformationen des an jedem Zahn angebrachten Halteteils auf der Grundlage einer Klebefläche des angebrachten Halteteils.

12. Verfahren nach Anspruch 11, wobei das Generieren des Korrekturbildes des Weiteren umfasst:
Vergleichen des Bildes des angebrachten Halteteils mit dem vorab gespeicherten orthodontischen Halteteilbild; und
Extrahieren eines orthodontischen Halteteilbildes, das mit dem Bild des angebrachten Halteteils in dem vorab gespeicherten orthodontischen Halteteilbild übereinstimmt.

13. Verfahren nach Anspruch 12, wobei das Generieren des Korrekturbildes des Weiteren das Platzieren des übereinstimmenden orthodontischen Halteteilbildes im selben Abschnitt wie das Bild des an jedem Zahn angebrachten Halteteils auf der Grundlage der Positionsinformationen des angebrachten Halteteils umfasst.

## Revendications

1. Dispositif informatique (12) qui comporte un ou plusieurs processeurs (14), et une mémoire configurée pour stocker un ou plusieurs programmes exécutés par le ou les processeurs (14), le dispositif informatique (12) comprenant :
une partie d'acquisition d'image (110) configurée pour acquérir une image orale d'un intérieur de la cavité buccale d'un patient cible à l'aide d'un scanner 3D intra-oral ;
un analyseur d'image (130) configuré pour générer une image de dent et une image d'un boîtier fixé à une pluralité de dents à travers l'image orale obtenue ;
un correcteur d'image (140) configuré pour faire correspondre l'image du boîtier fixé et une image de boîtier orthodontique incluse dans des informations de boîtier orthodontique préenregistrées et pour générer une image de correction pour l'image orale sur la base de l'image de dent et de l'image de boîtier orthodontique correspondante ; et
un générateur de chemin (150) configuré pour générer un chemin de courbure d'un fil sur la base de l'image de correction.

2. Dispositif informatique selon la revendication 1, dans lequel le générateur de chemin calcule une position d'une rainure de boîtier orthodontique dans l'image de correction sur la base des informations de boîtier orthodontique correspondantes et génère un point virtuel au niveau de la position calculée de la rainure de boîtier orthodontique pour générer le chemin de courbure du fil.

3. Dispositif informatique selon la revendication 2, dans lequel le chemin de courbure du fil génère au moins deux points virtuels ou plus pour chaque dent, et
les points virtuels sont deux points ou plus générés dans des limites gauche et droite de la position calculée de la rainure de boîtier orthodontique.

4. Dispositif informatique selon la revendication 1, dans lequel l'analyseur d'image génère une image de chaque dent dans la cavité buccale sur la base de l'image orale, génère une image d'un boîtier fixé à chaque dent, et génère des informations de position du boîtier fixé à chaque dent sur la base d'une surface adhésive du boîtier fixé.

5. Dispositif informatique selon la revendication 4, dans lequel le correcteur d'image compare l'image du boîtier fixé avec l'image de boîtier orthodontique préenregistrée et extrait une image de boîtier orthodontique qui correspond à l'image du boîtier fixé dans l'image de boîtier orthodontique préenregistrée.

6. Dispositif informatique selon la revendication 5, dans lequel le correcteur d'image place l'image de boîtier orthodontique correspondante dans la même partie que l'image du boîtier fixé à chaque dent sur la base des informations de position du boîtier fixé.

7. Dispositif informatique selon la revendication 6, dans lequel le correcteur d'image fait correspondre l'image de boîtier orthodontique correspondante et l'image du boîtier fixé à l'aide d'un algorithme itératif du point le plus proche (ICP).

8. Procédé de génération d'un chemin de courbure d'un fil de redressage de dents, qui est effectué dans un dispositif informatique (12) comportant un ou plusieurs processeurs (14) et une mémoire pour stocker un ou plusieurs programmes exécutés par le ou les processeurs (14), le procédé comprenant :
l'obtention (S602) d'une image orale d'un intérieur de la cavité buccale d'un patient cible à l'aide d'un scanner 3D intra-oral ;
la génération (S604) d'une image de dent et d'une image d'un boîtier fixé à une pluralité de dents au moyen de l'image orale obtenue ; et
la mise en correspondance (S606) de l'image du boîtier fixé et d'une image de boîtier orthodontique incluse dans des informations de boîtier orthodontique préenregistrées et la génération d'une image de correction pour l'image orale sur la base de l'image de dent et de l'image de boîtier orthodontique correspondante, et
la génération (S608) d'un chemin de courbure d'un fil sur la base de l'image de correction.

9. Procédé selon la revendication 8, dans lequel la génération du chemin de courbure du fil comporte en outre :
le calcul d'une position d'une rainure de boîtier orthodontique dans l'image de correction sur la base des informations de boîtier de correction correspondantes ;
la génération d'un point virtuel au niveau de la position calculée de la rainure de boîtier orthodontique pour générer le chemin de courbure du fil ; et
la sortie du chemin de courbure généré du fil.

10. Procédé selon la revendication 9, dans lequel le chemin de courbure du fil génère au moins deux points virtuels ou plus pour chaque dent, et
les points virtuels sont deux points ou plus générés dans des limites gauche et droite de la position calculée de la rainure de boîtier orthodontique.

11. Procédé selon la revendication 8, dans lequel la génération de l'image de dent et de l'image du boîtier fixé à la pluralité de dents comporte en outre :
la génération d'une image de chaque dent dans la cavité buccale sur la base de l'image orale ;
la génération d'une image d'un boîtier fixé à chacune des dents ; et
la génération d'informations de position du boîtier fixé à chaque dent sur la base d'une surface adhésive du boîtier fixé.

12. Procédé selon la revendication 11, dans lequel la génération de l'image de correction comporte en outre :
la comparaison de l'image du boîtier fixé avec l'image de boîtier orthodontique préenregistrée ; et
l'extraction d'une image de boîtier orthodontique qui correspond à l'image du boîtier fixé dans l'image de boîtier orthodontique préenregistrée.

13. Procédé selon la revendication 12, dans lequel la génération de l'image de correction comporte en outre le placement de l'image de boîtier orthodontique correspondante dans la même partie que l'image du boîtier fixé à chaque dent sur la base des informations de position de l'image du boîtier fixé.
